# EUROPEAN PATENT SPECIFICATION

(11) **EP 0 385 156 B1**
(45) Date of publication and mention of the grant of the patent: **12.01.1994**
(21) Application number: 90102554.4
(22) Date of filing: 09.02.1990
(51) Int. Cl.: A61M 15/00

(54) **Disposable inhaler with pre-pierced capsule**
Inhalator zur einmaligen Verwendung mit vorperforierter Kapsel
Inhalateur jetable avec capsule préperforée

(30) Priority: 23.02.1989 IT 1954089
(43) Date of publication of application: 05.09.1990
(73) Proprietor: PH&T S.r.l., 20145 Milano (IT)
(72) Inventor: Valentini, Luigi, I-20145 Milan (IT); Ceschel, Giancarlo, I-20145 Milan (IT)
(74) Representative: Luksch, Giorgio, Dr.-Ing.

(56) References cited:
- US-A- 3 669 113

## Description

This invention relates to inhalers, ie those devices for administering medicaments in finely divided form contained in capsules by inhalation.

In particular, reference should be made to the inhaler described in US-A-4,069,819, one of the two inventors of which is also one of the two inventors of the present invention. Said patent describes an inhaler of overall cylindrical shape comprising a body enclosing an axially extending nebulization chamber.

A capsule containing the medicament in finely divided form is placed in the chamber. The capsules of this type have an overall cylindrical shape with their two ends shaped as a spherical cap. The nebulization chamber is of overall cylindrical shape and has a substantially larger cross-section than the cross-section of the capsule to be placed in the chamber. This latter communicates with the outside through air inlet apertures in the chamber wall at or in proximity to one end of the chamber and shaped to generate a swirling air flow through the chamber during inhalation. The chamber also communicates with the outside through air discharge apertures provided in the other spherical cap-shaped end of the chamber and opening into an axial discharge duct.

The inhaler is also provided with a piercing device for piercing the casing of the capsule which has previously been inserted into the nebulization chamber. The piercing device is arranged to produce two or more holes in that spherical cap-shaped end of the capsule facing the air inlet apertures.

When the capsule has been pierced the user brings the inhaler up to his nostril or mouth and breathes in. On breathing in, the particular arrangement of the air inlet holes generates within the nebulization chamber a swirling air flow which causes the pierced capsule to undergo such movements as to cause the medicament in finely divided form to escape through its holes, so that the powder is entrained outwards by said air flow. This latter passes through said discharge apertures and flows along the subsequent discharge duct until it reaches either the oral cavity or the nasal cavity of the patient, as the case may be.

However, the aforesaid inhaler has certain drawbacks. In this respect it has been found that in a large number of cases the emptying of the capsule is irregular and incomplete, with consequent difficulties in administering the finely divided medicament.

Further examples of prior art inhalers are given in US-A-3 669 113.

The object of the present invention is to substantially simplify the structure of disposable inhalers of the aforesaid type.

Said object is attained by the disposable inhaler according to the present invention, in which the medicament is contained in a conventional capsule pierced before being inserted into the inhaler, comprising a hollow body delimiting an overall cylindrical, axially extending nebulization chamber, one end of the chamber being closed by a wall and the other end of the chamber by a perforated dome-shaped baffle through which the inhalation occurs, the body being provided with apertures for allowing air to enter the chamber during inhalation, the capsule being inserted into the nebulization chamber during the production of the inhaler, means being provided for preventing escape of the medicament through the holes of the pre-pierced capsule until the inhaler is used, said means for preventing escape being movable from a first position wherein the medicament is prevented from escaping from the capsule, to a second position wherein the inhaler is ready for use, said means for preventing escape consisting of a cup which embraces the spherical cap-shaped terminal part of the capsule in which the holes are provided thus closing said holes, characterized in that the buffle is disposed inside the hollow body, the apertures are provided in the lateral wall of the hollow body near the wall which closes the chamber, and the cup is fixed to spokes which pass through the apertures, the spokes being themselves fixed to an esternal operating sleeve for moving the cup away from its first position.

The invention will be more apparent from the description of some embodiments thereof given hereinafter by way of example only. In said description reference is made to the accompanying drawings in which:
Figure 1 is an axial longitudinal section through a first embodiment of a disposable inhaler according to the invention, ready to be used by axially squeezing it, the right half of the figure showing the inhaler as sold, whereas the left half of the figure shows it ready for use;
Figure 2 is an axial longitudinal section through a second embodiment of the invention ready to be used by traction, the right half of the figure again showing the inhaler as sold; and
Figure 3 is an axial longitudinal section through a third embodiment of the invention of the type used by traction, the left half of the figure showing the inhaler ready for use.

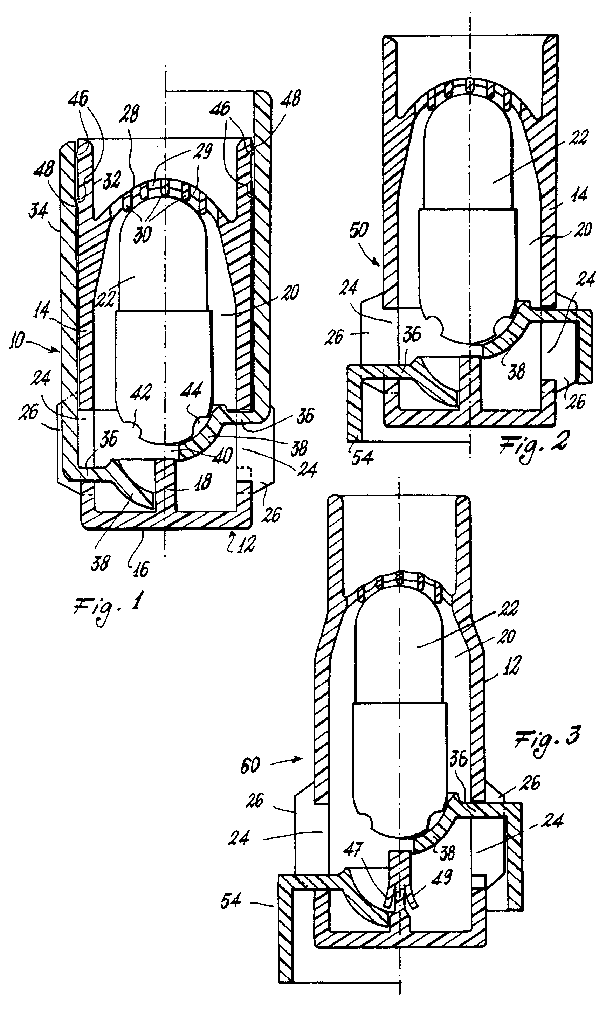

From Figure 1 it can be seen that the disposable inhaler 10 comprises a body 12 of overall cylindrical shape comprising a tubular side wall 14 and a base wall 16, fixed to the tubular wall 14. A coaxial cylindrical element 18 extends upwards from the base wall 16. The body 12 encloses a nebulization chamber 20 arranged to receive an ovoid capsule 22 of well known type containing the medicament in finely divided form to be administered by inhalation. The nebulization chamber 20 can be made to communicate with the outside through the air inlet slits 24 provided in the side wall 14 of the cylindrical body 12. For simplicity, the figures show only those slits corresponding with the section plane. These are of the type described in US-A-4,069,819 (to which reference should be made), these slits also being externally delimited by fins 26.

The nebulization chamber is lowerly of cylindrical shape and is upperly tapered to connect to a perforated baffle 28 of dome shape, from the inner surface of which there project pegs 30 parallel to the axis of the inhaler 10, their purpose being as explained in the aforesaid copending patent application. The remaining upper part of the body 12 forms a discharge channel 32 for the air laden with the medicament in finely divided form.

An operating sleeve 34 provided with a certain number of spokes 36 extending inwards through said slits 24 can slide in both directions on the outer surface of the cylindrical body 12. To the inner end of said spokes 36 there is fixed a coaxial cup 38 having a coaxial hole 40 through which the cylindrical element 18 can slide. The cup 38 has a shape which mates with the shape of the spherical cap end of the pre-pierced capsule 22. When the cup 38 is in the position shown to the right of Figure 1, it closes the holes 42, 44 in the capsules 22 to prevent the medicament in finely divided form contained in the capsule 22 from escaping through said holes. The cup 38 can move together with the operating sleeve 34, the travel of which is limited by the presence of the spokes 36 which slide in the slits 24.

Consequently by moving the sleeve 34 the cup 38 is able to move between the two outer positions shown in Figure 1, the position shown to the right corresponding to that at the time of sale of the inhaler (the cup 38 closing the holes 42, 44 in the capsule 22), whereas the position shown to the left is that in which the inhaler is ready for use. In reality, in Figure 1 the capsule 22 is shown in the situation corresponding to the upper end position of the sleeve 34 and thus of the cup 38, the true position which the capsule assumes when the sleeve is in its lower end position not being shown for simplicity.

Suitable means are provided to prevent undesired movement of the sleeve 34 and to lock it in the position for use. As the inhaler is usually of plastic material, said means can for example consist of transverse ribs 46 and 48 provided on the outer lateral surface of the body 12 and on the corresponding inner lateral surface of the sleeve 34 respectively. These ribs project from the relative surface in such a manner as to mutually interfere, so that to move the sleeve 34 from one of its two end positions it is necessary for the rib 48 of the sleeve 34 to pass over the relative rib 46 of the body 12. Other means can obviously be used to obtain the same result, such as the elastic tongues 47, 49 shown in Figure 3, the purpose of which is to lock the cup 38 in the position for use.

As is apparent, to use the inhaler of Figure 1 it is necessary only to exert an axial squeezing action on it to withdraw the cup 38 from the perforated base of the capsule 22. After that, inhalation can proceed. To ensure separation of the capsule 22 from the cup 38 an extractor means 18 is provided which when the inhaler is ready for inhalation keeps the capsule in the optimum position for initiating its precession movement as soon as inhalation commences.

Figure 2 shows a modification of the disposable inhaler according to the invention in which those parts which remain identical to those of Figure 1 are indicated by the same reference numerals.

The only substantial difference compared with the embodiment of Figure 1 is that to arrange the inhaler 50 for use a pulling action must be exerted on it. More precisely, the sleeve 54, which extends downwards from the spokes 36, has a greater diameter than the sleeve of Figure 1 because it has to slide over the fins 26 of the cylindrical body 12. To arrange the inhaler 50 for use it is necessary only to grip both the lateral surface of the body 12 and the lateral surface of the sleeve 54 with the fingers and axially pull them apart until they stop. Again in this case suitable means can be provided to ensure that a certain force is required to move the sleeve from its end positions, as in the case of the inhaler 10 of Figure 1, or means for locking the cup in its lower end position, as in the case of the inhaler 60 of Figure 3.

If the inhaler is of the type for applying to a nostril it will have a narrower air discharge duct than those shown in Figures 1 and 2. The modified embodiment shown in Figure 3 represents an inhaler of this type, which is otherwise substantially identical to that of Figure 2, the modification obviously also being applicable to the inhalers of Figures 1 and 2.

In the case of Figures 2 and 3, suitable knurling can be applied to the lateral surface of the body 12 and sleeve 54 to facilitate gripping.

In all the aforedescribed embodiments of the inhaler according to the invention it is necessary for the cylindrical body 12 to be constructed in at least two pieces, one of which for example forms the upper part of the lateral wall 14 as far as the lower edge of the slits 24, and the other forms the base wall 16 with the relative remaining part of the lateral wall 14. The two pieces are then joined together by gluing, ultrasonic welding or similar fixing methods.

However, before doing this the cup 38 must be placed inside by inserting the relative spokes 36 into the corresponding slits 24, which are still open at their bottom. In the illustrated embodiments the cup can be in the form of a substantially elastic membrane fixed to the spokes, this providing excellent closure of the capsule holes when the membrane is in, its upper position. The embodiment comprising the membrane can be formed either by simply making the cup with fairly thin walls or by gluing a suitable membrane to a ring carried by said spokes.

In all the illustrated embodiments the spokes which support the cup and connect it to the external operating sleeve pass through the slits through which air enters the nebulization chamber.

These spokes can instead pass through separate apertures provided for this purpose in the lateral wall of the cylindrical body.

The cup 38 also functions as a centering means for the pre-pierced capsule 22, which after insertion into the nebulization chamber 20 will not necessarily lie coaxially therein. For this purpose the cup 38 has its upper edge conveniently flared to facilitate centering of the capsule when the cup is moved into the position in which it closes the capsule holes.

Besides its use in administering common medicaments by inhalation, the inhaler according to the invention has proved particularly useful in administering calcitonin, parathyroid hormone and gonadorelin, each vehicled with mannitol or other suitable vehicles.

## Claims

1. A disposable inhaler (10;50;60) for administering medicaments in finely divided form, the medicament being contained in a conventional capsule (22) pierced before being inserted into the inhaler, comprising a hollow body (12) delimiting an overall cylindrical, axially extending nebulization chamber (20), one end of the chamber (20) being closed by a wall (16) and the other end of the chamber (20) by a perforated dome-shaped baffle (28) through which the inhalation occurs, the body (12) being provided with apertures (24) for allowing air to enter the chamber (20) during inhalation, the capsule (22) being inserted into the nebulization chamber (20) during the production of the inhaler, means (38) being provided for preventing escape of the medicament through the holes (42,44) of the pre-pierced capsule (22) until the inhaler is used, said means (38) for preventing escape being movable from a first position wherein the medicament is prevented from escaping from the capsule (22), to a second position wherein the inhaler is ready for use, said means for preventing escape consisting of a cup (38) which embraces the spherical cap-shaped terminal part of the capsule (22) in which the holes (42,44) are provided thus closing said holes, characterized in that the baffle (28) is disposed inside the hollow body (12), the apertures (24) are provided in the lateral wall of the hollow body near the wall (16) which closes the chamber (20), and the cup (38) is fixed to spokes (36) which pass through the apertures (24), the spokes (36) being themselves fixed to an esternal operating sleeve (34) for moving the cup (38) away from its first position.

2. A disposable inhaler as claimed in Claim 1, characterised by comprising an extractor means (18) for ensuring that the capsule (22) separates from the means (38) which prevent escape of the medicament through the holes in the capsule when the sleeve (34) is operated.

3. A disposable inhaler as claimed in Claim 1 or 2, characterised by comprising means (46, 48, 47, 49) for preventing undesired movement of the sleeve (34) and to lock it in the position for use.

## Patentansprüche

1. Wegwerfbarer Inhalator (10;50;60) für das Verabreichen von Medikamenten in feinverteilter Form, bei welchem das Medikament in einer konventionellen Kapsel (22) enthalten ist, die vor dem Einsetzen in den Inhalator durchbohrt wird, der einen Hohlkörper (12) umfaßt, der eine insgesamt zylindrische sich axial erstreckende Vernebelungskammer (20) begrenzt, wobei ein Ende der Kammer (20) durch eine Wand (16) und das andere Ende der Kammer (20) durch eine durchbohrte domförmige Prallwand (28) verschlossen wird, durch welche das Inhalieren auftreten kann, wobei der Körper (12) mit Öffnungen (24) versehen ist, damit Luft während des Inhalierens in die Kammer (20) eintreten kann, wobei die Kapsel (22) während der Herstellung des Inhalators in die Vernebelungskammer (20) eingesetzt wird, ein Mittel (38) vorgesehen ist, um ein Entweichen des Medikaments durch die Löcher (42,44) der vorher durchbohrten Kapsel (22) solange zu verhindern, bis der Inhalator verwendet wird, wobei dieses Mittel (38) von einer ersten Stellung, in der verhütet wird, daß das Medikament aus der Kapsel (22) entweichen kann, in eine zweite Stellung verschieblich ist, in der der Inhalator gebrauchsbereit ist, wobei dieses Mittel zum Verhindern eines Entweichens aus einer Glocke (38) besteht, welche den kugelkappenförmigen Endteil der Kapsel (22) überdeckt, in welchem die Löcher (42,44) vorgesehen sind und folgliche jene Löcher verschließt, **dadurch gekennzeichnet**, daß die Prallwand (28) im Innern des Hohlkörpers (12) angeordnet ist, die Öffnungen (24) in der Seitenwand des Hohlkörpers in der Nähe der Wand (16), welche die Kammer (20) verschließt, vorgesehen sind und die Glocke (38) an Speichen (36) befestigt ist, welche durch die Öffnungen (24) hindurchgehen, wobei die Speichen (36) selbst an einer externen Betätigungsmuffe (34) befestigt sind, um die Glocke (38) weg von ihrer ersten Stellung zu bewegen.

2. Wegwerfbarer Inhalator nach Anspruch 1, **dadurch gekennzeichnet**, daß er ein Extraktionsmittel (18) umfaßt, um zu gewährleisten, daß sich die Kapsel (22) von dem Mittel (38) trennt, welches ein Entweichen von Medikament durch die Bohrungen in der Kapsel verhütet, wenn die Muffe (34) betätigt wird.

3. Wegwerfbarer Inhalator nach Anspruch 1 oder 2, **dadurch gekennzeichet**, daß er Mittel (46, 48, 47, 49) für eine unerwünschte Bewegung der Muffe (34) und zum Verriegeln derselben in der gebrauchsbereiten Stellung umfaßt.

## Revendications

1. Inhalateur jetable (10, 50, 60) servant à administrer des médicaments sous forme finement divisée, le médicament étant contenu dans une capsule standard (22) percée avant d'être introduite dans l'inhalateur, comprenant un corps creux (12) délimitant une chambre de vaporisation (20) essentiellement cylindrique et allongée dans le sens axial, l'une des extrémités de la chambre (20) étant fermée par une cloison (16) et l'autre extrémité de la chambre (20) par un déflecteur perforé en forme de dôme (28) à travers lequel s'effectue l'inhalation, le corps (12) étant muni d'ouvertures (24) permettant à l'air de pénétrer dans la chambre (20) pendant l'inhalation, la capsule (22) étant introduite dans la chambre de vaporisation (20) lors de la fabrication de l'inhalateur, un moyen (38) étant prévu pour empêcher que le médicament ne s'échappe par les orifices (42, 44) de la capsule (22) préalablement percée, jusqu'à ce que l'inhalateur soit utilisé, ledit moyen (38) servant à empêcher l'échappement pouvant se déplacer d'une première position dans laquelle le médicament ne peut pas s'échapper de la capsule (22), à une seconde position dans laquelle l'inhalateur est prêt à l'emploi, ledit moyen servant à empêcher l'échappement étant constitué par une coupelle (38) qui enserre la partie terminale en forme de capuchon sphérique de la capsule (22) dans laquelle sont prévus les trous (42, 44), obturant ainsi lesdits orifices, caractérisé en ce que le déflecteur (28) est disposé à l'intérieur du corps creux (12), en ce que les ouvertures (24) sont disposées dans la paroi latérale du corps creux à proximité de la cloison (16) qui ferme la chambre (20), et en ce que la coupelle (38) est fixée à des rayons (36) qui passent au travers des ouvertures (24), les rayons (36) étant eux-mêmes fixés à un manchon externe d'entraînement (34) permettant de dégager la coupelle (38) de sa première position.

2. Inhalateur jetable selon la revendication 1, caractérisé en ce qu'il comprend un moyen d'extraction (18) prévu pour s'assurer que la capsule (22) est bien désolidarisée du moyen (38) servant à empêcher l'échappement du médicament par les trous de la capsule, lorsque le manchon (34) est actionné.

3. Inhalateur jetable selon l'une ou l'autre des revendications 1 ou 2, caractérisé en ce qu'il comprend des moyens (46, 47, 48, 49) empêchant le mouvement inopiné du manchon (34) et le bloquant dans sa position d'utilisation.
